# EUROPEAN PATENT APPLICATION

(11) **EP 4 611 307 A1**
(43) Date of publication of application: **03.09.2025**
(21) Application number: 25160585.3
(22) Date of filing: 27.02.2025
(51) Int. Cl.: H04L 9/00

(54) **TREATMENT DEVICE, SECURE TREATMENT SYSTEM AND METHOD**

(30) Priority: 29.02.2024 US 202463559816 P; 19.12.2024 US 202418986860
(71) Applicant: Sanmai Technologies, PBC, Sunnyvale, CA 94087 (US)
(72) Inventor: DAFT, CHRISTOPHER, SUNNYVALE, CA, 94087 (US)
(74) Representative: Araujo, Daniel

(57) **Abstract**

The invention relates to a treatment device (101), a secure treatment system (100, 200), and a method for securely authorizing and executing treatments using a blockchain network (170). The treatment device (101) comprises at least one processor (140), a secure enclave (150) for cryptographic security, and an application configured to retrieve a treatment list from the blockchain network (170), request authorization for a selected treatment, and retrieve a corresponding treatment recipe for execution. The system (100, 200) comprises at least one treatment device (101), a blockchain network (170), and a control client device (230) configured to execute a blockchain transaction with the blockchain network (170) via a control smart contract (245). Each treatment device (101) is configured to request authorization to execute a selected treatment from the treatment list, wherein the system (100, 200) determines whether authorization is granted before providing the corresponding treatment recipe. The method ensures secure and verifiable treatment execution while preventing unauthorized modifications.

## Description

### TECHNICAL FIELD

The present invention relates to treatment devices and systems, particularly medical and wellness devices incorporating security mechanisms to prevent unauthorized access or tampering. The invention further relates of a method of authorizing a treatment device to execute a selected treatment.

### PRIOR ART

Medical and wellness treatment devices are increasingly being used in both clinical and non-clinical settings for administering various treatments to users. Such devices may include transcranial ultrasound systems, electrostimulation devices, and other therapeutic hardware designed to provide targeted stimulation or treatment based on predefined treatment protocols.

A critical challenge in the deployment of such devices is ensuring the integrity and security of the treatments administered. Unauthorized modifications, tampering, or unauthorized use of treatment devices can pose significant risks to patient safety, treatment efficacy, and regulatory compliance. For example, an unauthorized user may attempt to modify treatment parameters beyond safe limits, or a device could be subjected to physical tampering to bypass security controls.

Ensuring transparency and verifiability of treatments remains a challenge. Patients, physicians, and regulatory bodies often require immutable records of treatment data to confirm that prescribed treatments are administered correctly and without unauthorized modifications. Conventional centralized databases or cloud storage solutions do not provide tamper-proof guarantees, as data can be altered by administrators or compromised by cyberattacks.

Therefore, there is a need for an improved treatment device providing enhanced security, transparency, and user authentication. The present invention aims to overcome these limitations by proposing a treatment device and system that addresses that addresses the problem of ensuring secure and authorized treatment delivery in treatment devices and systems.

### SUMMARY OF THE INVENTION

A first aspect of the invention refers to a treatment device comprising a secure enclave, at least one processor configured to communicate with a blockchain network, and an application configured to be executed by the at least one processors of the treatment device.

The application comprises instructions that, when executed by the processor, enables the treatment device to interact securely with the blockchain network. The instructions of the application are configured to cause the at least one processor to generate a request for retrieving a treatment list from the blockchain network, wherein the treatment list comprises one or more treatments suitable for execution by the treatment device. Upon generating the request, the instructions are configured to cause the processor to retrieve the treatment list from the blockchain network and to subsequently request authorization to execute a selected treatment from the treatment list. If authorization is confirmed via the blockchain network (e.g. in response to receiving authorization from the blockchain network), the application causes the treatment device to retrieve from the blockchain network a treatment recipe corresponding to the selected treatment, allowing the device to execute the treatment as specified.

The treatment device may be suitable for providing a wellness treatment and/or a medical treatment to a user. Preferably, the treatment device may comprise (or may be connectable to) a treating hardware configured to provide a treatment to a user based on a treatment recipe. The treating hardware may be configured as a transcranial ultrasound hardware designed to provide ultrasound targeting and stimulation to a user. This configuration is broadly compatible with all embodiments of the invention.

The secure enclave may be interpreted as referring to a module (e.g. a hardware module) designed to provide cryptographic security and protect sensitive information from unauthorized access or tampering. The secure enclave may operate as a trusted execution environment, ensuring data security, cryptographic integrity, and resistance to tampering, particularly in the context of blockchain-enabled treatment devices. It may be configured to store a private key and/or a master seed.

In the context of the present invention, a private key may refer to a cryptographic key uniquely associated with a blockchain account and used to generate digital signatures for authenticating transactions, proving ownership of digital assets, and securing communications. The private key may be mathematically linked to a corresponding public key, allowing third parties to verify signed messages or transactions without revealing the private key itself. In the context of a secure enclave, the private key may be generated, stored, and utilized within a protected execution environment, ensuring that it remains confidential and resistant to unauthorized access, extraction, or tampering.

In the context of the present invention, a master seed may refer to a cryptographic root value from which multiple private keys can be deterministically derived using a predefined key derivation function, typically conforming to a hierarchical deterministic (HD) wallet structure (e.g. suitable for generating a large number of externally owned accounts, EOAs). The master seed may enable secure key management and account recovery by allowing a user to regenerate all associated private keys from a single securely stored seed. In a secure enclave implementation, the master seed is protected within an isolated execution environment to prevent unauthorized access, modification, or extraction.

The at least one processor (e.g. microprocessors) of the treatment device may be part of a control block. The control block may comprise a System-on-Chip (SoC) integrating one or more microprocessors. Thus, the processor may form part of an SoC. The control block (e.g. the at least one processor) may be configured to control the overall operation of the treatment device.

The treatment device is configured to communicate with a blockchain network, also referred to as a blockchain database. To enable such communication, the treatment device may comprise a communication module, which is broadly and transversally compatible with all embodiments of the invention. The communication module allows the processor to exchange data with the blockchain network, ensuring secure, immutable, and verifiable interactions and/or transactions.

The treatment device may be implemented as a single ASIC (Application Specific Integrated Circuit) or may be implemented as a group of discrete devices integrated using a PCB or similar.

In some embodiments, the device may further comprise an anti-tampering module, also referred to as an anti-tampering block, which may include one or more sensors configured to detect a variation in an environmental and/or operational condition of the treatment device. Such variations may be indicative of a tampering attempt on the treatment device, allowing the system to take appropriate protective actions. The anti-tampering module may enhance the security and integrity of the device by detecting unauthorized access or manipulation, thereby preventing potential disruptions or security breaches.

The treatment device may further comprise a protective enclosure, also referred to as a housing, which may be configured to protect the secure enclave and/or the at least one processor. The housing may serve as a physical security barrier, preventing unauthorized access to critical components of the device. In such cases, the one or more sensors of the anti-tampering module (also referred to as the anti-tampering block) may comprise one or more sensors configured to detect potential tampering attempts by monitoring variations in environmental conditions within the protective enclosure (these sensors are also referred to as physical detection intrusion sensors). Preferably, these sensors may comprise: one or more sensors configured to detect variations in temperature or pressure within the protective enclosure that exceed a predetermined threshold, which may indicate unauthorized physical interference with the treatment device; and/or one or more light sensors configured to detect light exposure within the protective enclosure, which may indicate an attempt to open the protective enclosure or otherwise gain unauthorized access to its internal components.

In some embodiments, the anti-tampering module may include sensors configured to detect variations in voltage and/or frequency that deviate from respective predefined operational ranges of the treatment device. Such variations may indicate an attempt to interfere with the electrical operation of the treatment device, such as by injecting unauthorized signals or modifying the power supply to bypass security mechanisms. These types of sensors are combinable with the protective enclosure and/or with the previously described sensors configured to detect tampering attempts to the protective enclosure.

To further enhance security, the at least one processor may be configured to take protective actions when an indication of tampering on the treating device is detected by the anti-tampering module (e.g. by any of the above-described sensors). In particular, the processor may be configured to perform at least one of the following actions: erasing the secure enclave (e.g. preventing unauthorized access to sensitive cryptographic data and ensuring the integrity of secure operations); activating an alarm (e.g. which may generate an alert to notify a user or a remote system of the detected tampering attempt); and/or shutting down the treatment device (e.g. to prevent its operation until proper authorization is re-established, thereby mitigating potential security breaches).

The fact that the processor is configured to perform at least one of these protective actions should be interpreted as meaning that the treatment device may be configured to perform only one of the actions, or may be configured to perform any combination of two of these actions, or the three actions. For example, the treatment device may be configured to (i) erase the secure enclave and activate an alarm, (ii) erase the secure enclave and shut down the treatment device, or (iii) activate an alarm and shut down the treatment device. In some embodiments, the application may comprise instructions which, when executed by the at least one processor upon an indication of tampering received from the anti-tampering module, may cause the at least one processor to perform at least one of the aforementioned protective actions.

Further, the anti-tampering block may operate in conjunction with hardware encryption accelerators (which may be integrated into the anti-tampering block), which may be configured to encrypt high-bandwidth data, such as targeting information, in real-time to ensure data confidentiality and security.

These security mechanisms may ensure that the treatment device remains protected against unauthorized access, physical tampering, and electrical interference, thereby maintaining the integrity of its interaction with the blockchain network and the secure execution of treatments.

The secure enclave of the treatment device may be configured to store a private key suitable for signing and decrypting messages sent to and received from the treatment device (e.g. messages and/or blockchain transactions with the blockchain network). This cryptographic functionality enables secure communication and ensures the integrity of transactions within the system. Preferably, the secure enclave may be further configured to prevent the retrieval or revelation of its contents, thereby enhancing the security of sensitive cryptographic information and protecting it from unauthorized access or tampering.

To ensure that only authorized users can operate the treatment device, the device may further comprise a user authentication module configured to verify the identity of a user before allowing operation of the treatment device. The authentication module may perform identity verification by comparing an information provided by the user (e.g., a password and/or biometric information) with a corresponding authentication information (e.g., the correct password and/or the biometric information of an authorized user). The authentication information may be stored in the secure enclave. This secure authentication process ensures that only authorized users can access and operate the treatment device.

In some embodiments, the authentication module may comprise at least one biometric sensor (e.g. such as a camera for facial recognition and/or a fingerprint reader) for sensing biometric information of the user. The at least one biometric sensor may be configured to store and/or compare biometric data within the secure enclave, ensuring that biometric authentication is performed in a secure and tamper-resistant environment. By leveraging biometric authentication, the treatment device enhances security and ease of use, ensuring that access is restricted to authorized individuals.

The treatment device may further comprise a blockchain client configured to control the communication between the at least one processor and the blockchain network. In operation, the blockchain client may rely on the optional connecting module. The blockchain client enables secure interaction with the blockchain by facilitating the retrieval and validation of treatment data, authorization processes, and execution of blockchain transactions. Preferably, the blockchain client may be configured to sign communications with the blockchain using a private key stored within the secure enclave, ensuring transaction integrity and cryptographic security. This private key may be the same private key previously described as being suitable for signing and decrypting messages sent to and received from the treatment device, thereby maintaining a unified cryptographic security framework within the system.

A second aspect of the invention refers to a system (also referred to as secure treatment system) comprising a blockchain network (which may be configured as a p2p blockchain network), at least one treatment device (which may be configured to run a light client wallet application such as WallETH or Status), one or more smart contracts (comprising at least a control smart contract), and a control client device, wherein the system is configured to securely manage and execute treatment-related transactions (e.g. including blockchain transactions) within a blockchain infrastructure.

The blockchain network of the system may be configured for securely managing transactions related to treatment data (also referred to as treatment-related data or treatment data specification). The treatment data comprises: one or more treatment lists (e.g. each treatment list comprising a list of treatments suitable for being executed by each treatment device) and treatment recipes with instructions for executing each treatment; wherein the treatment data may further comprise a prize/cost associated with each treatment. The one or more smart contracts (including the control smart contract) are configured to be deployed on the blockchain network to perform blockchain transactions.

The at least one treatment device of the system is configured to communicate with the blockchain network. The at least one treatment device may be preferably configured according to any of the embodiments previously described for the first aspect of the invention, meaning it may incorporate security features such as a secure enclave, anti-tampering mechanisms, user authentication, and a blockchain client. Each treatment device is configured to interact with the control smart contract to request and retrieve a treatment list from/via the blockchain network. The treatment list comprises one or more treatments that are suitable for execution by the respective treatment device.

In operation, a treatment device may be configured to request authorization from/via the blockchain network to execute a selected treatment from the treatment list. The selection of treatment may be made manually by a user of the treatment device or may be made automatically by the treatment device based on predefined preferences of use. The system is configured for determining whether the treatment device has authorization to execute the selected treatment. If authorization is confirmed (e.g. upon verification via the one or more smart contracts deployed on the blockchain network hat the at least one treatment device is authorized to execute the selected treatment), the blockchain network provides the treatment device with the corresponding treatment recipe, allowing the device to execute the selected treatment based on the retrieved treatment instructions. In other words, upon a determination via the blockchain network that the at least one treatment device has authorization to execute the selected treatment, the corresponding treatment recipe is provided to the treatment device via the blockchain network. In some embodiments, one or more smart contracts deployed in the blockchain may be configured to determine whether the at least one treatment device has authorization to receive this recipe.

Accordingly, the system may be configured to determine whether the treatment device has authorization to execute the selected treatment by verifying one or more authorization conditions defined in the blockchain network, preferably enforced by one or more smart contracts configured to be deployed on the blockchain network. Optionally, the authorization conditions may include, for example, at least one of: (i) verifying that the treatment device requesting authorization to execute a selected treatment is eligible to perform the selected treatment based on stored treatment-related data; (ii) verifying that a required payment, if applicable, has been successfully processed via a blockchain transaction (e.g. via a control smart contract); and/or (iii) verifying that a prescription authorization or other prior authorization requirement has been met (e.g. such as an approval recorded in a physician smart contract). If the authorization is successfully verified, the blockchain network provides the treatment recipe of the selected treatment to the treatment device.

Additionally, the control client device is configured to execute a blockchain transaction with the blockchain network via the control smart contract to allow modifying the treatment data stored in the blockchain network, wherein the treatment data comprises at least a treatment list and a treatment recipe for each treatment included in the treatment list, and optionally includes additional information, such as a cost/prize for each treatment. By interacting with the blockchain network, the control client device ensures that the treatment-related data remains secure, immutable, and verifiable, preventing unauthorized modifications while enabling controlled updates to the treatment information stored on the blockchain.

This system architecture ensures that treatment execution is conducted in a transparent, tamper-resistant, and verifiable manner, leveraging the blockchain network to enhance data integrity, security, and decentralized authorization control.

The treatment data, also referred to as treatment-related data or data specifying treatments, may be stored on the blockchain or on a data availability source controlled by the blockchain, such as Celestia, Avail, or EigenDA. The treatment data includes a treatment list, which specifies the treatments compatible with a given treatment device, and a treatment recipe for each treatment. A treatment recipe refers to a set of instructions and/or parameters stored on the blockchain, defining how the treatment device should execute a particular treatment. By storing treatment recipes on the blockchain, the system ensures that treatment parameters remain secure, immutable, and verifiable, preventing unauthorized modifications or misuse.

An important advantage of the system of the seconds aspect of the invention is that the treatment device does not store any information related to the treatments (e.g. such as the list of treatments or the treatments recipes). In contrast, the system is configured such that, every time that the treatment device is operated for executing a selected treatment, the corresponding treatment recipe of this selected treatment is obtained at that moment.

In some embodiments, the treatment data stored in the blockchain network may further comprise a cost associated with each treatment of the treatment list. The cost may define the amount of digital currency, tokens, or other blockchain-based assets required to execute a given treatment on a treatment device. Each treatment device of the system may be configured to execute a blockchain transaction with the control smart contract to process a payment corresponding to the cost of the selected treatment. Preferably: the feature that the system is configured for determining whether the treatment device has authorization to execute the selected treatment may comprise (e.g. require) determining by the control smart contract that the payment of the cost of the selected treatment has been successfully completed; and/or the system may be further configured such that, upon successful payment processing, the control smart contract is modified to reflect that the payment transaction has been successfully completed. This modification may ensure immutability and verifiability of payment records, thereby preventing unauthorized treatment execution or fraudulent activity. This modification may ensure immutability and verifiability of payment records, thereby preventing unauthorized treatment execution or fraudulent activity. Thus, the control smart contract may be configured to verify that the payment has been successfully completed before granting authorization for the treatment execution.

This blockchain-based payment mechanism enhances the security and transparency of treatment transactions by ensuring that only fully paid and authorized treatments can be executed by the treatment device. The payment verification process may also integrate with other authorization requirements, such as physician approval stored in a physician smart contract, ensuring compliance with medical and regulatory frameworks.

In some embodiments, the blockchain network may be configured such that the treatment data is stored in the control smart contract (which is configured to be deployed on the blockchain network). The control smart contract may act as a secure and immutable data structure deployed on the blockchain network, ensuring that treatment-related data remains tamper-proof, transparent, and verifiable. Storing treatment data within the control smart contract enables automated execution of predefined conditions and facilitates secure interactions between treatment devices, control client devices, and other authorized entities.

Preferably, the control client device may be further configured to modify the treatment data stored in the control smart contract. This modification may include updating one or more of: treatment lists, treatment recipes, and/or associated pricing data. Additionally, or complementarily, in some embodiments, the control client device may be configured to replace the control smart contract with an updated version containing updated treatment data. This update mechanism ensures that the system can adapt to changes in medical protocols, regulatory compliance, or newly developed treatments while maintaining the integrity and security of stored data.

To facilitate controlled access and authorized modifications, the control client device may be configured to be connected to a controlling organization externally owned account (EOA) - also referred to as control EOA. The control EOA is an independent blockchain account controlled by an entity (e.g., a manufacturer, regulatory authority, or medical institution) that is responsible for executing blockchain transactions of the control client device. Through this connection, the control client device may securely interact with the control smart contract to modify treatment-related data, ensuring that only authorized entities can make modifications within the blockchain network.

Additionally, in some embodiments, the control smart contract may comprise a control smart contract data module, which may be configured to store at least one of the following: a treatment list, specifying one or more treatments that are executable by each of the at least one treatment device; treatment recipes, which define the set of instructions and/or parameters specifying how each treatment should be executed by the treatment device; and pricing data, associated with the execution of treatments by the treatment devices (e.g. allowing blockchain-based payment processing as previously described). By structuring the control smart contract to include a dedicated data module, the system enhances the efficiency, organization, and accessibility of stored treatment-related data. This configuration ensures that treatment execution, authorization, and payment processing remain securely managed within the blockchain network while providing a flexible and updatable framework for long-term use.

In some embodiments, the system may further comprise a physician smart contract and a physician client device. The physician smart contract may be configured to be deployed on the blockchain network to perform blockchain transactions (e.g. transactions related to management of prescription authorizations for the execution of treatments by the treatment devices).

The physician client device may be configured to execute blockchain transactions via the physician smart contract to manage prescription authorizations for each of the at least one treatment devices to execute the one or more treatments of the treatment list. Thus, the physician client device enables healthcare professionals (e.g. physicians) to manage prescription authorizations, ensuring compliance with medical regulations and treatment safety requirements. The physician client device may be used to add, modify, or revoke prescription authorizations, thereby allowing a dynamically update of the prescription authorizations in the system.

Each treatment device within the system may be configured to interact with the physician smart contract to verify whether a selected treatment has prescription authorization before execution. In such cases, the previous feature related to the fact that the system is configured for determining whether the treatment device has authorization to execute the selected treatment may comprise (e.g. require) determining (e.g. verifying), via the physician smart contract, that the treatment device has prescription authorization for executing the selected treatment. This verification process ensures that unauthorized or unapproved treatments cannot be executed by the treatment device, thereby enhancing patient safety and treatment compliance.

The physician smart contract may further comprise a physician smart contract data module, which may be configured to store prescription authorization data (e.g. data related to the prescription authorizations). The prescription authorization data may indicate whether a selected treatment has been approved for execution by a treatment device. Preferably, the physician client device may be further configured to modify the prescription authorization data stored in the physician smart contract, and/or to replace the physician smart contract with an updated physician smart contract with updated prescription authorization data.

In some embodiments, the physician client device may be configured to connect to a physician externally owned account (EOA), which may serve as a blockchain account controlled by an authorized healthcare provider. The physician EOA (which may be regarded as a part of the blockchain network of the system of the second aspect of the invention) may be configured to enable secure authentication and transaction execution, ensuring that only verified professionals (e.g. medical professionals, such as physicians) can manage prescription authorizations. The physician EOA may be used to execute blockchain transactions that create, update, or revoke prescription authorizations within the physician smart contract.

By integrating a physician smart contract and physician client device, the system provides a secure, decentralized, and verifiable method for managing prescription-based treatment execution, ensuring that only authorized treatments are performed while maintaining regulatory compliance and data integrity.

In some embodiments, the blockchain network of the system may be configured as an Ethereum-based network or a Solana-based network. These blockchain platforms provide a secure, decentralized, and verifiable infrastructure for managing treatment execution, authorization, and transaction processing within the system.

In an embodiment, the blockchain network may be configured as an Ethereum-based network, wherein the system is configured such that, upon determination that the treatment device has authorization to execute a selected treatment, this authorization is provided via smart contract code (e.g. via the control smart contract and/or the physician smart contract) running on an Ethereum Virtual Machine (EVM). The EVM enables secure execution of smart contracts, ensuring that treatment authorization and transaction processing are immutable, transparent, and verifiable. Preferably, the Ethereum-based blockchain network may be configured as a layer-2 chain, which is designed to settle blockchain transactions on Ethereum. Layer-2 networks enhance scalability and reduce transaction costs by processing transactions off-chain while maintaining the security of the Ethereum main chain. A layer-2 chain settling on Ethereum refers to a scalability solution that operates off-chain while periodically settling transactions on the Ethereum main chain. This configuration reduces transaction costs and improves system efficiency, ensuring that the blockchain-based authorization and payment processes remain fast, secure, and cost-effective.

In another embodiment, the blockchain network may be configured as a Solana-based network, wherein the system is configured such that, upon determination that the treatment device has authorization to execute a selected treatment, this authorization is provided via smart contract code running (e.g. via the control smart contract and/or the physician smart contract) on a Solana Virtual Machine (SVM). The Solana blockchain is designed for high-speed transaction processing with low latency, making it well-suited for handling real-time authorization requests and blockchain-based payment transactions within the system.

An advantage of this blockchain configurations is that they are designed to be decentralized and that users (e.g. patients) are not required to reveal their identities to use the blockchain network, so that privacy of the user is guaranteed. Blockchain networks have numerous validators, whose function is to propose and evaluate the correctness of new blocks, and geographically dispersed. For example, in early 2024, the Ethereum-based network had over 600,000 computers acting as validators. It also had 6,000 full nodes enforcing network rules. This level of decentralization makes it difficult even for state actors to disrupt the orderly functioning of this blockchain. In traditional cloud-based solutions, server failure is an ever-present concern, even with fail-over arrangements. In contrast, the decentralized blockchain approach, where the smart contracts (e.g. the control smart contract and/or the physician smart contract) run in parallel on every (Ethereum or similar) full node, is far more reliable.

Another advantage of the system according to the second aspect of the invention is that the blockchain network stores transaction data immutably, such that it is not possible to alter transactions after finalizing the transaction chain. Additionally, while smart contract code and past executions remain immutable, certain smart contract data may be updated if the contract is designed to allow state changes.

A third aspect of the invention refers to a method (e.g. a computer implemented method) of authorizing a treatment device to execute a selected treatment within a blockchain-based system, ensuring secure, verifiable, and decentralized authorization for treatment execution. The method comprises a sequence of blockchain-based interactions that allow a treatment device to obtain authorization and treatment execution parameters from the blockchain network.

The method comprises generating, by the treatment device, a request for receiving a treatment list from the blockchain network. The treatment list includes one or more treatments suitable for execution by the treatment device. The treatment device retrieves the treatment list from the blockchain network and subsequently submits a request for authorization to execute a selected treatment from the retrieved list.

The method further comprises determining, via the blockchain network (e.g. via the one or more smart contracts configured to be deployed on the blockchain network), whether the treatment device has authorization to execute the selected treatment. This determination may be conducted via smart contract logic (e.g. via the previously described control smart contract and/or physician smart contract), ensuring that only eligible and authorized treatments are executed. Upon confirmation of authorization, the treatment device retrieves a treatment recipe from the blockchain network, which defines the set of parameters and instructions required for executing the selected treatment.

Preferably, the step of determining whether the treatment device is authorized to execute a selected treatment may comprise verifying at least one of the following conditions:
- Determining, preferably by the control smart contract, that a payment of the cost of the selected treatment has been successfully completed (this ensures that the execution of a treatment is financially validated through a blockchain-based transaction before authorization is granted); and/or
- Determining, preferably by a physician smart contract, that the treatment device has prescription authorization to execute the selected treatment (this ensures compliance with medical and regulatory requirements, restricting treatment execution to authorized medical prescriptions stored in the blockchain network).

This blockchain-based authorization process enhances the security, transparency, and verifiability of treatment execution, ensuring that only authorized treatments can be executed by treatment devices.

The present invention, according to three aspects of the invention, utilizes features of blockchain technology, such as those available in the Ethereum blockchain, though it may also be implemented on other smart contract-compatible blockchain platforms. Blockchain networks typically support two types of accounts: externally owned accounts (EOAs) and smart contract accounts.

In this context of the present invention, an externally owned account (EOA) is normally controlled by a private key and is limited to sending and receiving transactions. It cannot store arbitrary data but may include relevant information in a transaction's Data field. The EOA (e.g. a physician EOA and/or a control EOA) may also hold balances of digital assets, such as Ether or other tokens used within the blockchain network.

In contrast, and also in the context of the present invention, a smart contract account is normally configured to store and process arbitrary data. When a smart contract is executed as part of a transaction, the Data field of the transaction may contain encoded function calls and parameters for the smart contract. The smart contract processes this data and updates its state (e.g. in the corresponding data module) accordingly. These state updates remain persistent within the blockchain, ensuring that the data remains accessible as long as the blockchain network exists. Alternatively, smart contract-related data may be stored on blockchain-managed data availability providers, such as Celestia, Avail, or EigenDA, enhancing scalability and efficiency.

Smart contracts arbitrate policy enforcement and transactional interactions among treatment devices, physicians, patients, and treatment records. The characteristics of smart contracts contribute to the security and transparency objectives of the system. A smart contract is a self-executing system in which the terms of the agreement are directly embedded in software code. The code and the agreement therein exist in smart contracts operating on a decentralized blockchain network, allowing transactions and agreements to be executed securely without reliance on a central authority, legal system, or external enforcement mechanism.

Once deployed, a smart contract executes automatically upon fulfillment of predefined conditions, enforcing the associated contractual terms, without the need for an intermediary to intervene. Since smart contracts are immutable once written to the blockchain, they may noy be altered post-deployment, thereby ensuring high levels of security and reliability. The blockchain nodes of the blockchain network validate each transaction associated with the smart contract, ensuring transparency and tamper resistance.

Smart contracts are typically implemented using platform-specific programming languages, such as Solidity in the case of Ethereum. Once deployed, each contract is assigned a unique blockchain address. The contract autonomously executes its predefined terms upon the occurrence of trigger events (e.g. pre-set conditions), such as receiving a payment or authorization request. This enables automation of processes, execution of transactions, and enforcement of system rules, including: processing payments for wellness treatments, registering new treatment recipes, and/or authorizing a treatment device to perform a specific treatment.

The smart contract of the present invention is configured to maintain a record of all published treatment recipes. These data are stored within the smart contract's state (e.g. the respective data module of the corresponding smart contract) or, potentially, as "blobs" on a layer-2 data availability layer, such as an off-chain storage mechanism. While a smart contract is immutable once deployed, system updates can be facilitated by deactivating an existing smart contract and redirecting transactions to a newly deployed contract. This approach ensures that system updates remain verifiable on the blockchain while preserving transparency and integrity. Any modifications to the system, including changes in ownership of the controlling entity or updates to corporate policies, remain publicly auditable.

### BRIEF DESCRIPTION OF THE DRAWINGS

Fig. 1 shows treatment device 101 according to the first aspect of the invention operating in system 100.
Fig. 2 illustrates a secure treatment system 200 according to embodiments of the second aspect of the invention compatible with system 100.
Fig. 3 shows an exemplary method 300, where the treatment device 101 is used in a system 100, 200 according to the second aspect of the invention as a wellness device.
Fig. 4 shows an exemplary method 400, where the treatment device 101 is used in a system 100, 200 according to the second aspect of the invention as a prescription treatment device.
Fig. 5 shows an exemplary method 500 used in a system 100, 200 according to the second aspect of the invention to update the treatment list or change the pricing.

### DETAILED DESCRIPTION OF THE DRAWINGS

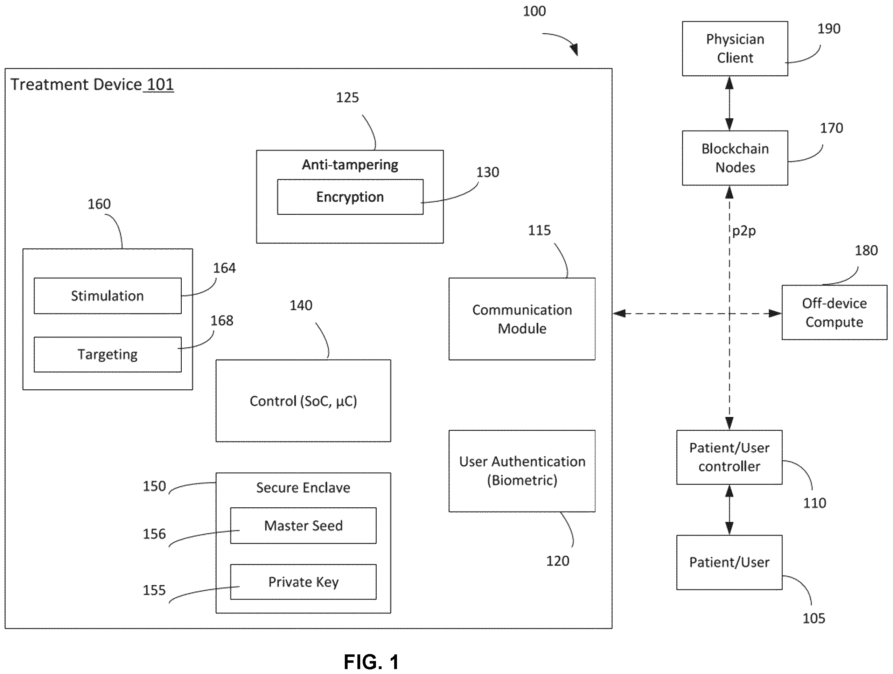

Figure 1 illustrates a treatment device 101 according to the first aspect of the invention operating within a system 100 that provides anti-tampering, security, and transparency features. The treatment device 101 comprises at least one processor 140 (depicted as part of an optional control block), a secure enclave 150, an optional anti-tampering module 125, an optional user authentication module 120, and an optional communication module 115. The treatment device 101 (e.g. the at least one processor) is configured to communicate with a blockchain network 170 (depicted as block representing blockchain nodes 170), preferably via the optional communication module 115 and a blockchain client.

The secure enclave 150 (which may be configured as a hardware module that ensures cryptographic security by storing sensitive information) is depicted according to the optional configuration in which comprises (e.g. stored therein) a private key 155 and a master seed 156. The private key is utilized for signing and decrypting messages (e.g. for blockchain transactions) exchanged between the treatment device 101 and the blockchain network 170, while the master seed 156 enables a hierarchical deterministic HD wallet software to generate a large number of externally owned accounts (EOAs). The private key 155 and the master seed 156 may be written to the secure enclave 150 during manufacturing of the secure enclave 150. The secure enclave 150 may be configured to operate independently from the rest of the treatment device 101.The secure enclave 150 may be configured to encrypt messages which may be used outside of blockchain network 170 and may also be configured to decrypt messages sent to the treatment device 101 (which may have been encrypted with tis public key).

These capabilities are helpful for firmware and software updates to the treatment device 101. For example, updates cab be signed by a source of the software and encrypted with a public key of the treatment device 101. After the update has been completed, the treatment device 101 may sign a message confirming that the update has been completed.

The anti-tampering module 125 comprises one or more sensors configured to detect variations in environmental and operational conditions indicative of unauthorized tampering attempts. These sensors include at least one or more of: temperature and pressure sensors, voltage and frequency monitoring sensors, and light sensors capable of detecting exposure within an optional enclosure of the treatment device 101. Upon detection of a tampering attempt, the at least one processor 140 may trigger a security response, such as erasing the secure enclave 150, activating an alarm, or shutting down the treatment device 101 (e.g. in a way that can be reversed only at manufacturing premises where the treatment device 101 is manufactured).

Fig. 1 depicts an optional embodiment in which the anti-tampering module 125 comprises (or operates in conjunction with) hardware encryption accelerators 130, which may be configured to encrypt high-bandwidth data, such as targeting information (e.g. targeting information for a treating hardware configured as a transcranial ultrasound hardware for providing ultrasound stimulation to a user 105 on specific target areas of the head), in real-time to ensure data confidentiality and security.

The optional user authentication module 120 is configured to verify the identity of a user 105 before allowing operation of the treatment device 101. Fig. 1 shows the preferred embodiment in which the authentication module 120 comprises one or more biometric sensors for reading biometric information, which may be securely stored within the secure enclave 150 to ensure authentication integrity. The user authentication process ensures that only authorized individuals can access the treatment device 101.

The treatment device 101 is configured to retrieve treatment-related data from the blockchain network 170, which includes a treatment list specifying treatments executable by the device and corresponding treatment recipes. A selected treatment undergoes an authorization process, which may be managed via a control smart contract (not shown in Fig. 1) and/or an optional physician smart contract (not shown in Fig. 1). Upon successful authorization, the treatment device 101 retrieves the treatment recipe from the blockchain network 170 and is ready to execute the selected treatment.

The treating hardware 160, which is either integrated into or connectable to the treatment device 101, is configured to provide a selected treatment (which may be a medical treatment or a wellness treatment) to a user 105 based on the corresponding treatment recipe. In the preferred embodiment, the treating hardware 160 is a transcranial ultrasound hardware designed for ultrasound targeting 168 and stimulation 164.

The blockchain network 170 is configured as a decentralized infrastructure ensuring immutability, transparency, and security of treatment-related transactions. In some embodiments, the blockchain network 170 may be implemented as an Ethereum-based network or a Solana-based network, with smart contract execution taking place on an Ethereum Virtual Machine (EVM) or a Solana Virtual Machine (SVM), respectively. The treatment device 101 may employ a peer-to-peer (p2p) network for communication with the blockchain network 170.

Fig. 1 shows an optional patient/user controller 110 configured to allow a patient/user 105 to interact (preferably remotely) with the treatment device 101. The patient/user controller 110 may include a display, input buttons, and other user interface elements to enable the user to select treatments, view authorization status, and receive treatment instructions. The patient/user controller 110 may be configured to be integrated as part of the treatment device 101 (e.g. as on-device UX -user experience- elements, such as buttons, switches and/or biometric sensors) or may be configured to be part of a separate device, such as an electronic device (e.g. a smartphone or similar devices). In some embodiments, the patient/user controller 110 may integrate biometric authentication features to further ensure that only authorized users can operate the treatment device 101. The patient/user controller 110 ensures an intuitive and user-friendly experience for individuals operating the treatment device 101.

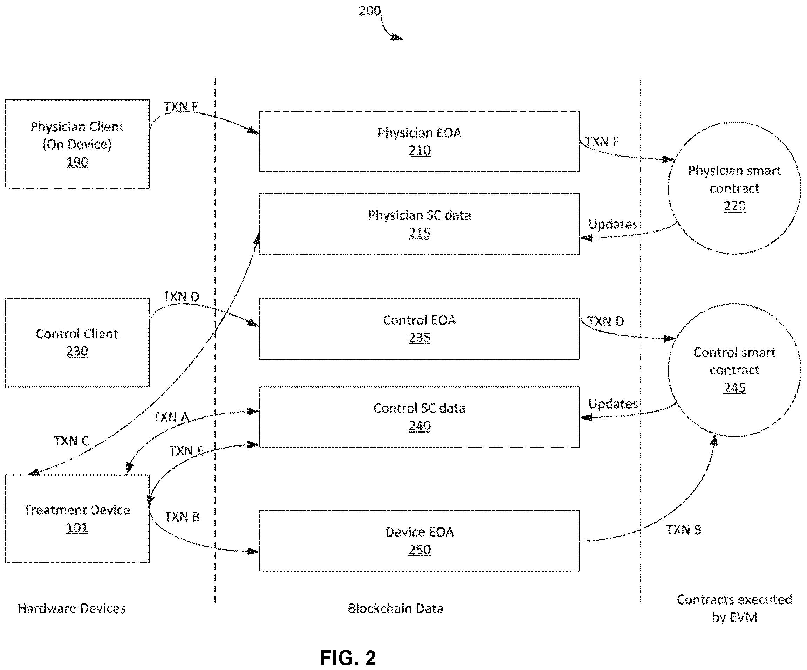

Fig. 1 also depicts an optional physician client interface 190 (e.g. integrated as part of a physician client device 190 as shown in Fig. 2) being integrated as part of the system 100. The physician client device 190 is configured to execute transactions within the blockchain network 170, enabling a healthcare professional to authorize (e.g. a physician), modify, or revoke prescription authorizations for executing treatments in the treatment devices 101 (e.g. to regulate which treatment devices are allowed to be executed by each treatment device 101). The physician client device 190 ensures compliance with medical regulations and enhances patient safety by restricting treatment execution to authorized users. This physician client device 190 may be implemented as a smartphone, tablet, or computer running a blockchain physician client application that allows secure interaction with the treatment device 101. The physician client device 190 may be configured to interact with a physician smart contract 220 (as shown in Fig. 2).

The treatment device 101 may be further configured to communicate with other devices 180 external to the treatment device, which may be configured as servers and/or off-device compute resources 180 independent not involved in blockchain transactions.

Although not shown in Fig. 1, the system 100 is also compatible with a control client device 230 (as the one shown in Fig. 2), which may be configured to interact with the blockchain network 170 via a control smart contract 245 to modify treatment data (e.g. treatment lists, treatment recipes, treatment costs, etc.). The control client device 230 enables updates to treatment lists, treatment recipes, and associated pricing data. Additionally, a physician client device 190 may be utilized to manage prescription authorization via transactions with the physician smart contract 220, ensuring that treatment execution complies with regulatory requirements.

The treatment device 101 and its associated system 100 enable secure, tamper-resistant, and verifiable interactions for treatment execution, leveraging blockchain technology to ensure data integrity, compliance, and patient safety.

Further, the treatment device 101 of Fig. 1 (which may be configured as a medical and/or wellness device) may be configured to generate personal health information (PHI) through its operation. For example, when the treating hardware 160 is configured as a transcranial ultrasound hardware, targeting information may be provided by the treatment device 101 to an off-device 180 computing engine, wherein this targeting information remains inaccessible to other entities/elements external to the system 100. The treatment device 101 may be configured to provide the PHI over a wireless link such as WiFi or Bluetooth, whose encryption may not be sufficiently secure to share the PHI. Thus, in those embodiments comprising the hardware encryption accelerators 130, this hardware 130 may be configured to encrypt the PHI before sending it over wireless links.

Fig. 2 illustrates a secure treatment system 200 (which is also compatible with the system 100 shown in Fig. 1) configured to provide tamper-resistant, verifiable, and decentralized management of treatment execution using blockchain technology. The system 200 comprises a treatment device 101, a control client device 230, an optional physician client device 190, a control smart contract 245, an optional physician smart contract 220, and a blockchain network 170 facilitating multiple transaction types. Referring to Fig. 2, three categories are shown: hardware, data on the blockchain network, and smart contract programs (e.g. executable by EVM or Ethereum Virtual Machine), which may also be stored on the blockchain network.

The blockchain network 170 of the system 200 may be configured for securely managing transactions related to treatment data (also referred to as treatment-related data or treatment data specification). The control smart contract is a blockchain-based contract configured to be deployed on the blockchain network 170 to perform blockchain transactions in the blockchain network 170, as described below in more detail.

The at least one treatment device 101 of the system 200 is configured to communicate with the blockchain network 170, being preferably configured according to any of the embodiments previously described for the first aspect of the invention (e.g. including the treatment device 101 of Fig. 1). Each treatment device 101 is configured to interact with the control smart contract 245 to request and retrieve a treatment list from/via the blockchain network 170. The treatment list comprises one or more treatments that are suitable for execution by the respective treatment device 170.

The control client device 230 is configured to execute a blockchain transaction with the blockchain network 170 via the control smart contract 245 to modify the treatment data stored in the blockchain network 170, wherein the treatment data comprises at least a treatment list and a treatment recipe for each treatment included in the treatment list, and optionally includes additional information, such as a cost/prize for each treatment. By interacting with the blockchain network 170, the control client device 230 ensures that the treatment-related data remains secure, immutable, and verifiable, preventing unauthorized modifications while enabling controlled updates to the treatment information stored on the blockchain.

Fig. 2 shows the preferred and optional configuration in which the treatment data further comprises a cost associated with each treatment of the treatment list. Each treatment device 101 of the system 200 may be configured to execute a blockchain transaction with the control smart contract 245 to process a payment corresponding to the cost of the selected treatment. Preferably, the system 200 may be configured such that, upon successful payment processing, the control smart contract 245 is modified to reflect that the payment transaction has been successfully completed. Thus, the control smart contract may be configured to verify that the payment has been successfully completed before granting authorization for the treatment execution.

The system 200 is configured for determining whether the treatment device has authorization to execute the selected treatment. If authorization is confirmed, the blockchain network 170 provides the treatment device 101 with the corresponding treatment recipe, allowing the treatment device 101 to execute the selected treatment based on the retrieved treatment instructions. Determining whether the treatment device 101 has authorization to execute the selected treatment may comprise verifying at least one of the following conditions: determining by the control smart contract 245 that a payment of the cost of the selected treatment has been successfully completed; and/or determining by a physician smart contract (as described below) that the treatment device 101 has prescription authorization to execute the selected treatment.

Fig. 2 shows the preferred and optional configuration in which the system 200 comprises a physician client device 190 and a physician smart contract 220. The physician smart contract 220 is configured to be deployed on the blockchain network 170 to perform blockchain transactions (e.g. transactions related to management of prescription authorizations for the execution of treatments by the treatment devices).

The physician client device 190 is configured to execute transactions with the blockchain network 170 via the physician smart contract 220 to manage prescription authorizations for treatments. Therefore, the physician client device 190 (together with the physician smart contract 220) enables a healthcare professional to add, modify, or revoke prescription authorizations for particular treatments to be executed in a particular treating device 101.

Accordingly, each treatment device 101 within the system 200 may be configured to interact with the physician smart contract 220 to verify whether a selected treatment has prescription authorization before execution. In such cases, the previous feature related to the fact that the system is configured for determining whether the treatment device has authorization to execute the selected treatment may comprise (e.g. require) determining (e.g. verifying), via the physician smart contract 220, that the treatment device 101 has prescription authorization for executing the selected treatment. This verification process ensures that unauthorized or unapproved treatments cannot be executed by the treatment device 101, thereby enhancing patient safety and treatment compliance.

The control client device 230 shown in Fig. 2 is configured to be connected to a controlling organization externally owned account, EOA (also referred to as control EOA 235, which is an optional feature of the embodiment). The control EOA 235 is an independent blockchain account controlled by an entity (e.g., a manufacturer, regulatory authority, or medical institution) that is responsible for executing blockchain transactions of the control client device 230. Through this connection, the control client device 230 may securely interact with the control smart contract 245 to modify treatment-related data, ensuring that only authorized entities can make modifications within the blockchain network.

Fig. 2 also shows the optional configuration in which the control smart contract 245 comprises a control smart contract data module 240. The control smart contract data module 240 may be configured to store at least one of the following: a treatment list, treatment recipes, and pricing data of the treatments.

Fig. 2 shows that the physician smart contract comprises a physician smart contract data module 215 (which is an optional feature of the embodiment), which is be configured to store prescription authorization data (e.g. data related to the prescription authorizations). The prescription authorization data indicate whether a selected treatment has been approved for execution in a treatment device 101.

In the embodiment of Fig. 2, the physician client device 190 comprises (or is deployed in connection to) an optional physician externally owned account (EOA) 210 which serves as a blockchain account controlled by an authorized healthcare provider. The physician EOA 210 (which may be regarded as a part of the blockchain network 170 of the system 200 of the second aspect of the invention) may be configured to enable secure authentication and transaction execution, ensuring that only verified professionals (e.g. medical professionals, such as physicians) can manage prescription authorizations. The physician EOA may be used to execute blockchain transactions that create, update, or revoke prescription authorizations within the physician smart contract.

Although Fig. 2 indicates that the contracts are executed by EVM (Etherum Virtual Machine), this is configuration is merely one the possible options, since, as it has been previously described, the invention is compatible with other configurations.

The system 200 supports multiple transaction types, which facilitate secure and transparent interactions between the treatment device 101, control client device 230, physician client device 190, and the blockchain network 170. These types of transactions are labelled in Fig. 2 as TXN followed by a letter and are defined below. Thus, the system 200 may be configured to execute the following transactions:
- TXN A: The treatment device 101 requests and retrieves a treatment list, treatment characteristics, and prices from the control smart contract 245.
- TXN B: The treatment device 101 executes a blockchain transaction to process a payment for a selected treatment.
- TXN C: The treatment device 101 verifies prescription authorization by querying the physician smart contract 220.
- TXN D: The control client device 230 modifies the control smart contract 245 to update treatment-related data, such as adding new treatments or adjusting pricing or updating recipes.
- TXN E: The treatment device 101 requests/retrieves (bidirectional arrow) a treatment recipe from the control smart contract 245 after authorization and payment verification.
- TXN F: The physician client device 190 executes a blockchain transaction to update prescription authorization data in the physician smart contract 220.

Transaction A, TXN A, is a query (commonly referred to as a "call") initiated by the treatment device 101 to the control smart contract 245 to request and retrieve a treatment list and optionally treatment characteristics and/or prices. This query does not modify the blockchain state and may be performed locally without requiring a transaction fee ("gas"). The treatment device 101 may execute this call using a lightweight blockchain client, which allows efficient querying of blockchain data without downloading entire blocks. The amount of gas required for such EVM activity depends on the complexity of the smart contract. In those embodiments using an Ethereum-based network, gas is denominated in "gwei," a unit which equals 10⁻⁹ Ether. The arrow linking the treatment device 101 and the control smart contract data module 240 indicates that the call may inquire about the state of the data module 240, which in this embodiment contains the list of available treatments, their characteristics, and pricing information.

Transaction B, TXN B, is a payment transaction executed by the treatment device 101 via the device externally owned account 250 (device EOA 250) to process the cost of a selected treatment. The treatment device 101 holds one or more EOAs, enabling it to interact with the blockchain network 170 securely. The transaction may include a Data field containing an identifier corresponding to the selected treatment. Upon receipt of the payment, the control smart contract 245 updates its state and issues a standard blockchain transaction receipt to the treatment device 101, with a "success" status code confirming that the selected treatment has been authorized for execution. Since this transaction alters the blockchain state, it incurs gas fees in addition to the treatment cost.

Transaction C, TXN, C is a verification query executed by the treatment device 101 to determine whether a prescribed treatment has been authorized. The treatment device 101 queries the physician smart contract 220 to check whether a prescription authorization exists for the selected treatment. Like Transaction A, this transaction is a "call" that does not modify the blockchain state and therefore does not require gas fees. The query ensures that only authorized prescription-based treatments can be executed by the treatment device 101.

Transaction D is an update transaction executed by the control client device 230 to modify treatment-related data stored in the control smart contract 245. This transaction allows for the addition of new treatments, modification of treatment characteristics, adjustment of pricing, or updating of treatment recipes. Since this transaction alters the state of the blockchain, it is executed via the controlling organization externally owned account 235 (Control EOA 235) and requires gas fees. Importantly, while the transaction updates the smart contract's state, all previous states remain immutably stored on the blockchain, ensuring full traceability. This immutable record allows authorized entities (such as a block explorer or a node of the blockchain) to verify that no unauthorized modifications or tampering have occurred within the system 200.

Transaction E is a retrieval query executed by the treatment device 101 to request and retrieve a treatment recipe from the control smart contract 245 after authorization and payment verification.

The treatment device 101 queries the control smart contract data module 240 to obtain the treatment recipe (e.g. the execution parameters) corresponding to the selected treatment. This query ensures that the treatment device 101 retrieves only authorized and valid treatment recipes. Like Transactions A and C, this transaction is a "call" that does not alter the blockchain state and does not require gas fees.

Transaction F is a state-changing transaction executed by the physician client device 190 to update prescription authorization data stored in the physician smart contract 220. The physician client device 190 interacts with the blockchain network using the physician externally owned account 210 (physician EOA 210) to create, modify, or revoke a prescription authorization for a specific treatment. The transaction's Data field contains encoded function call information that updates the physician smart contract data module 215. The state change ensures that the prescription authorization data is securely stored on the blockchain, allowing treatment devices 101 to verify the authorization status of a prescribed treatment before execution.

The treatment device 101 within the systema 100, 200 may be used by user 105 either as a wellness device or for administering prescription-based treatments. The primary distinction between these two modes of operation is that prescription treatments require prior authorization from the physician client device 190. The interactions between the treatment device 101 and the blockchain nodes 170, as well as the transactions occurring within the blockchain network 170 are invisible to the user 105.

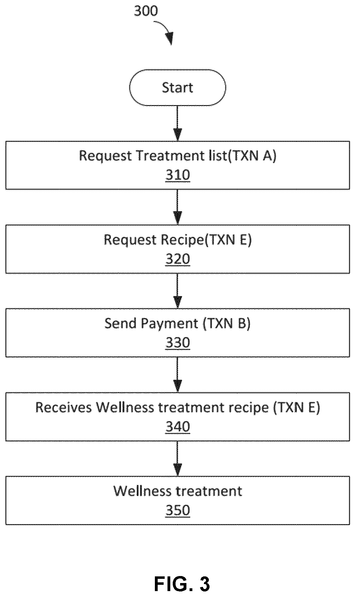

Figure 3 illustrates an exemplary method 300 for operating the treatment device 101 within the system 100, specifically when used as a wellness device (i.e. not requiring a prescription authorization). The method comprises a sequence of steps.

In Operation 310, the user 105 initiates a request to retrieve a list of available wellness treatments. The request is made via the treatment device 101 or via the patient/user controller 110, which optionally interacts with the user authentication module 120 of the treatment device 101 to verify the identity of the user 105. Authentication may be performed using biometric data or other authentication mechanisms. The treatment device 101 ensures that the user 105 meets all necessary requirements (e.g. legal, age, or usage restrictions) before proceeding. Once verified, the treatment device 101 communicates with the blockchain network via the optional communication module 115 to retrieve the wellness treatment list suitable for being executed by the treatment device 101 of the user 105. This request is executed as Transaction A, TXN A, a blockchain query that does not incur a transaction fee. The system 100 then returns the authorized wellness treatment list, which is displayed on the treatment device 101 or in the patient/user controller 110 as a selectable menu for user selection.

In Operation 320, the user 105 requests a treatment recipe of a selected (wellness) treatment r from the retrieved treatment list. The treatment recipe defines the operational parameters required for executing the selected treatment. For example, in the case of a treatment based on transcranial ultrasound stimulation, these parameters may include one or more of:
- Structures to be treated.
- Center frequencies of stimulation.
- Duration of stimulation waveforms.
- Quiescent periods between stimulation events.
- Number of stimulation events in the treatment session.
- Closed-loop parameters linking stimulation timing to measured biomarkers, such as electroencephalography or magnetoencephalography readings.

The request for retrieving the selected wellness treatment recipe in Operation 320 is executed as Transaction E, TXN E, a blockchain query that does not modify the blockchain state and does not incur transaction fees.

In Operation 330, the user 105 proceeds with the payment for the selected wellness treatment recipe. This payment is processed as Transaction B, TXN B, which requires interaction with the control smart contract 245. The payment may comprise two components: (i) a gas fee required to process the transaction within the blockchain network and (ii) a cost of the treatment itself.

In Operation 340, the treatment device 101 receives authorization to execute the requested wellness treatment. Upon successful payment verification via the control smart contract 245, the blockchain network transmits a confirmation message to the treatment device 101. This confirmation may include a transaction receipt confirming the authorization status. The patient/user controller 110 (or the treatment device 101 itself) may display a notification to the user 105 indicating that the requested wellness treatment is now available for execution. Additionally, detailed usage instructions, including information on device positioning and operational steps, may be presented to ensure proper application of the treatment.

In Operation 350, the user 105 positions the treatment device 101 correctly and proceeds with executing the requested wellness treatment. The treatment is performed according to the retrieved treatment recipe, ensuring precise application of the wellness procedure.

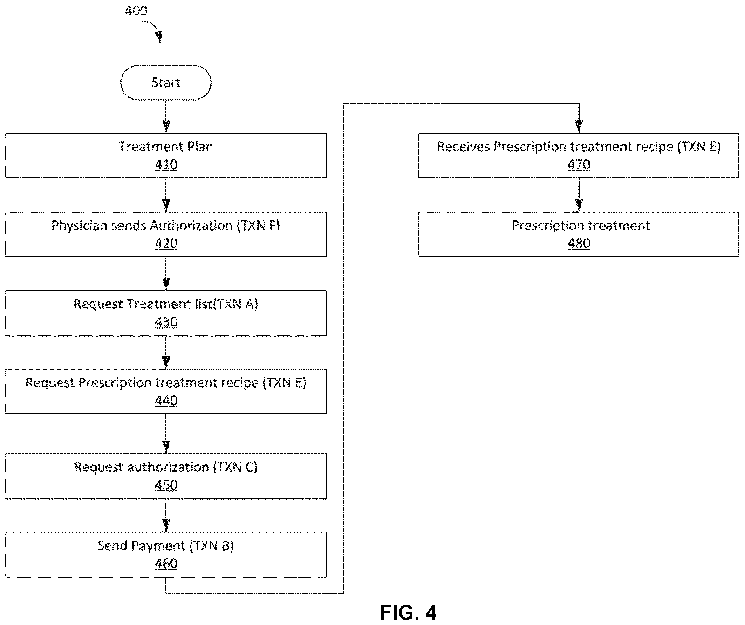

Figure 4 illustrates an exemplary method 400 for operating the treatment device 101 within the system 100 as a prescription treatment device. The method outlines a sequence of operations enabling secure and authorized execution of prescription-based treatments using blockchain technology.

In Operation 410, the patient or user 105 and a physician coordinate on a treatment plan. This process typically takes place outside the blockchain network. During this step, the physician or healthcare provider may generate a unique patient ID for the user 105, ensuring personalized treatment tracking and authentication.

In Operation 420, the physician utilizes a physician client device 190, such as a smartphone or computer, to submit an authorization transaction for the prescription treatment. This transaction is executed via the physician externally owned account 210 and sent to the physician smart contract 220. The transaction is recorded as Transaction F, TXN F, and requires only the gas fee necessary to interact with the physician smart contract 220, without involving any fund transfers.

In Operation 430, the user 105 requests a list of available prescription treatments. This request is initiated via the controller 110, which interacts with the user authentication module 120 of the treatment device 101 to verify the identity of the user 105. Authentication may involve biometric data or other authentication mechanisms. Once the user is authenticated, the treatment device 101 queries the blockchain network through the communication module 115 to retrieve the list of authorized prescription treatments. This request is executed as Transaction A, TXN A, a blockchain query that does not alter the blockchain state and does not incur transaction fees. The authorized prescription treatment list is returned and displayed on the controller 110, allowing the user 105 to select a treatment.

In Operation 440, the user 105 selects a prescription treatment recipe from the displayed list (although in some embodiments, the selection may be automatically made by the treatment device 101; e.g. if there is only one authorized treatment available). The treatment recipe includes the specific operational parameters necessary for executing the treatment (which, in the case of a treatment based on transcranial ultrasound stimulation, these parameters may correspond to those previously disclosed for Fig. 3.

The request to retrieve the selected prescription treatment recipe is executed as Transaction E, TXN E, a blockchain query that does not modify the blockchain state and does not incur transaction fees.

In Operation 450, the user 105 requests authorization for the selected prescription treatment recipe. The treatment device 101 queries the physician smart contract 220 to verify that the authorization issued in Operation 420 is valid. This verification is recorded as Transaction C, TXN C, and may be executed as a blockchain query without modifying the blockchain state or incurring transaction fees.

In Operation 460, the user 105 submits payment for the selected prescription treatment recipe. The payment transaction is executed via Transaction B, TXN B, and requires interaction with the control smart contract 245. The payment may comprise two components: (i) a gas fee required to process the transaction within the blockchain network and (ii) a cost of the prescription treatment. Upon successful processing of the payment, the treatment device 101 may receive a payment receipt.

In Operation 470, the treatment device 101 queries the physician smart contract 220 to receive final authorization for executing the requested prescription treatment. Upon successful verification, the controller 110 displays a notification to the user 105 confirming that the requested prescription treatment is now available. Additionally, detailed usage instructions, including information on device positioning and operational steps, may be provided to ensure proper application of the treatment.

In Operation 480, the user 105 correctly positions the treatment device 101 and proceeds with executing the requested prescription treatment. The treatment is performed according to the retrieved treatment recipe, ensuring precise application of the procedure while maintaining regulatory compliance and security.

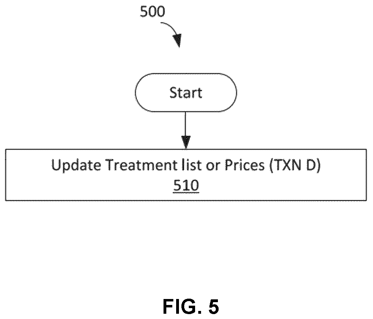

Figure 5 illustrates an exemplary method 500 implemented within the systems 100, 200 to facilitate updates to the treatment list, modify treatment pricing, or execute administrative adjustments to the behaviour of the overall system as reflected in the control smart contract data module 240. The method ensures secure and immutable modifications through blockchain transactions, maintaining system integrity and transparency.

In Operation 510, a controlling organization submits a transaction via its externally owned account 235 (control EOA 235) to the control smart contract 245, specifying the required changes. These changes may include updates to the treatment list, modifications to treatment characteristics, adjustments to pricing, or the addition of new treatment recipes. This transaction is executed as Transaction D, TXN D, ensuring that all modifications are securely recorded within the blockchain network. The updates specified in Transaction D, TXN D, may apply to both wellness and prescription-based treatments. The blockchain network processes these changes by executing the control smart contract (e.g. within the Ethereum Virtual Machine (EVM) or an equivalent blockchain execution environment). Because this transaction only modifies the blockchain state without transferring funds, the only cost incurred is the gas fee required to execute the control smart contract 245 operations.

Once the transaction is confirmed, the updated treatment data becomes available for retrieval by treatment devices 101 and other authorized system components, ensuring that all modifications are securely reflected in the blockchain ledger. This method guarantees that treatment execution remains compliant with the latest authorized data while preserving the integrity of historical treatment records.

By leveraging blockchain technology, the method 500 ensures that administrative changes to the system 100 are conducted transparently and verifiably, preventing unauthorized modifications while maintaining regulatory compliance and operational efficiency.

The ethical behavior of the controlling organization may be ensured through various governance structures implemented within the system. In one example, a decentralized autonomous organization (DAO) with on-chain governance may be utilized to manage decision-making within the blockchain-based framework. Alternatively, the controlling organization may adopt a traditional corporate governance structure, such as a Public Benefit Corporation or a non-profit entity, to ensure transparency and regulatory compliance.

A prime goal of the present invention is to enforce the immutability of treatment recipes, thereby ensuring that treatment parameters cannot be altered without detection. To facilitate transparency, the system enables third parties, including regulatory bodies and authorized users, to verify whether a treatment recipe has been modified using standard blockchain access tools such as web-based blockchain explorers or blockchain client applications. However, in certain cases, treatment recipes may require encryption to protect proprietary information while still ensuring verifiability. In such embodiments, the recipes may be stored in an encrypted format within the control smart contract data module 240. Even if encrypted, any unauthorized modifications to the encrypted data would remain detectable.

Referring to Figure 2, to support this functionality, the private key 155 stored within the secure enclave 150 of the treatment device 101 may be utilized to decrypt the published treatment information. The encryption process is independent of blockchain operations and may use a public/private key pair or a shared secret key for decryption.

In some implementations, encryption may be applied on a per-device basis, where a distinct encrypted treatment recipe is stored for each treatment device 101 within the control smart contract data module 240. This method may be combined with low-cost, off-chain immutable storage solutions, such as the storage provide by IPFS or other Layer 2 data availability layer storage. Alternatively, a single encrypted copy of the treatment recipe may be stored, with the same private key 155 preloaded into the secure enclave 150 of each authorized treatment device 101, allowing decryption at the device level.

To further enhance security, time-based one-time passwords (TOTP) may be employed to mitigate the risk of unauthorized access to encrypted data. This approach utilizes an algorithm that ensures any password sent remains valid only for a predefined period. In such cases, the control client device 230 of the controlling entity would periodically update the control smart control data module 240 of the control smart contract 245 each time the one-time password is refreshed. The secret algorithm responsible for generating and updating the TOTP would be securely shared between the control client device 230 and the treatment device 101. Decryption of the treatment recipe would occur entirely within the secure enclave 150, preventing unauthorized access by the at least one processor 140 (which may be configured as a system-on-chip, SoC) or other system components.

An additional security measure is derived from the manner in which smart contracts are deployed on the blockchain. Unlike plaintext code, smart contracts (e.g. the control smart contract 245 and/or the physician smart contract 220) may be stored and executed in a compiled bytecode format within the Ethereum Virtual Machine (EVM) or an equivalent blockchain execution environment. The compilation process provides an additional layer of security, as EVM bytecode differs significantly from traditional object code generated for physical processors. This characteristic makes it more difficult for an adversary to reverse-engineer the functionality of the smart contracts 220 and 245, further enhancing the security of the system.

Aspects of the present disclosure may be implemented as a system, method, or computer program product. Accordingly, implementations of the present invention may be realized in the form of a hardware-based system, a software-based system (including firmware, resident software, microcode, or other software components), or a combination of hardware and software elements. Any hardware and/or software techniques, processes, functions, components, engines, modules, or systems described herein may be implemented using dedicated circuitry, a combination of circuits, or as a computer-implemented process executed on suitable hardware.

Furthermore, aspects of the present disclosure may be embodied as a computer program product stored on a computer-readable medium comprising instructions executable by a processor to perform one or more of the disclosed methods. The term "computer-readable medium" refers to any tangible storage medium capable of storing instructions that can be executed by a computing device. Examples of such storage media include, but are not limited to, magnetic storage devices (e.g., hard drives), optical storage devices (e.g., CDs, DVDs), semiconductor memory devices (e.g., RAM, ROM, Flash memory, EPROM), electronic circuits, and other physical storage elements suitable for storing executable instructions.

Aspects of the present disclosure are further described with reference to flowcharts and/or block diagrams that illustrate methods, systems, and computer program products according to various embodiments. Each block within a flowchart or block diagram may represent a module, segment, or set of instructions of a computer program that, when executed by a processor, perform one or more specified functions. These blocks may be implemented using software instructions executed by a general-purpose computer, a specialized computing device, an application-specific integrated circuit (ASIC), a field-programmable gate array (FPGA), or another form of dedicated hardware.

It should be understood that the operations described in the flowcharts and block diagrams may be executed in the order presented or in an alternative sequence, including parallel execution where applicable. Additionally, certain blocks may be omitted or combined depending on the implementation requirements.

While the embodiments described herein illustrate specific implementations of the invention, it will be appreciated that modifications, variations, and alternative configurations may be devised without departing from the scope of the invention, which is defined by the claims.

## Claims

1. A treatment device (101) comprising:
a secure enclave (150);
at least one processor (140) configured to communicate with a blockchain network (170);
an application comprising instructions which, when the application is executed by the at least one processor, cause the at least one processor to at least:
generate a request for receiving a treatment list from the blockchain network (170), wherein the treatment list comprises a list of one or more treatments suitable for being executed by the treatment device (101);
retrieve the treatment list from the blockchain network (170);
request authorization from the blockchain network (170) to execute in the treatment device (101) a selected one of the one or more treatments from the list of treatments; and
in response to receiving authorization from the blockchain network (170), cause the treatment device (101) to retrieve from the blockchain network (170) a treatment recipe of the selected treatment for being executed by the treatment device (101);
wherein preferably the treatment device (101) comprises, or is connectable to, a treating hardware (160) configured to provide a treatment to a user based on the treatment recipe, wherein more preferably the treating hardware is configured as a transcranial ultrasound hardware for providing ultrasound stimulation to a user.

2. The device of claim 1, further comprising an anti-tampering module (125) comprising one or more sensors configured to detect a variation of an environmental and/or operational condition of the treatment device (101) indicative of a tampering attempt on the treatment device (101).

3. The device of claim 2, wherein the treatment device comprises a protective enclosure configured to protect the secure enclave (150) and/or the at least one processor (140), and wherein preferably the one or more sensors of the anti-tampering module (125) comprise:
one or more sensors configured to detect a variation of temperature or pressure within the protective enclosure exceeding a predetermined threshold; and/or
one or more light sensors configured to detect light exposure within the protective enclosure.

4. The device of claim 2 or 3, wherein the one or more sensors of the anti-tampering module (125) comprise one or more sensors configured to detect variations in voltage and/or frequency that deviates from respective predefined operational ranges of the treatment device (101), wherein said variations are indicative of an attempt to interfere with electrical operation of the treatment device (101).

5. The device of any of claims 2 to 4, further configured such that, when the at least one processor receives an indication of tampering from the anti-tampering module (125), the at least one processor (140) is configured to perform at least one of: erasing the secure enclave (150); activating an alarm; or shutting down the treating device (101).

6. The device of any of the preceding claims, wherein the secure enclave (150) is configured to store a private key (155) for signing and decrypting of messages from and to the treating device (101) respectively, wherein preferably the secure enclave (150) is configured to prevent a retrieval or revelation of any contents of the secure enclave (150).

7. The device of any of the preceding claims, further comprising a user authentication module (120) configured to verify identity of a user before allowing operation of the treatment device (101) by comparing information provided by the user with corresponding authentication information, preferably stored in the secure enclave (150);
wherein preferably the authentication module (120) comprises at least one biometric sensor configured to read biometric information of a user, and wherein the authentication information is configured as biometric information.

8. The device of any of the preceding claims, further comprising a blockchain client configured to control the communication between the at least one processor (140) and the blockchain network (170), wherein preferably the blockchain client is configured to sign communication with the blockchain using a private key stored in the secure enclave (150) for ensuring transaction integrity.

9. A secure treatment system (100, 200) comprising:
a blockchain network (170);
one or more smart contracts (220, 245) configured to be deployed on the blockchain network (170) to perform blockchain transactions, the one or more smart contracts (220, 245) comprising at least a control smart contract (245);
at least one treatment device (101) configured to communicate with the blockchain network (170), wherein the at least one treatment device (101) is preferably according to any of the preceding claims; and
a control client device (230) configured to execute a blockchain transaction with the blockchain network (170) via the control smart contract (245) to modify treatment data stored in the blockchain network (170), wherein the treatment data comprises: a treatment list of one or more treatments executable by each of the at least one treatment device (101), and a treatment recipe for each of the one or more treatments of the treatment list;
wherein each treatment device (101) of the at least one treatment device (101) is configured to interact with the control smart contract (245) to request and retrieve from the blockchain network (170) the treatment list;
wherein each treatment device (101) of the at least one treatment device (101) is further configured to request authorization from the blockchain network (170) to execute a selected treatment from the treatment list, wherein, upon a determination via the one or more smart contracts (220, 245) deployed on the blockchain network (170) that the at least one treatment device (101) has authorization to execute the selected treatment, the blockchain network (170) provides the treatment recipe of the selected treatment to the treatment device (101).

10. The system (100, 200) of claim 9, wherein the treatment data further comprises a cost for each treatment of the treatment list, and wherein each treatment device (101) of the at least one treatment device (101) is further configured to execute a blockchain transaction with the control smart contract (245) to process a payment of the cost of the selected treatment to the system (100, 200);
wherein preferably:
determining that the treatment device (101) has authorization to execute the selected treatment requires determining by the control smart contract (245) that the payment of the cost of the selected treatment has been successfully completed; and/or
the system (100, 200) is further configured such that, upon successful completion of the blockchain transaction to process the payment, the control smart contract (245) is modified to reflect the completion of the successful completion of said blockchain transaction.

11. The system (100, 200) of claim 9 or 10, wherein the blockchain network (170) is configured such that the treatment data is stored in the control smart contract (245);
wherein preferably the control client device (245) is further configured to modify the treatment data stored in the control smart contract (245), and/or to replace the control smart contract (245) with an updated control smart contract (245) with updated treatment data.

12. The system (100, 200) of any of claims 9 to 11, wherein:
the control client device (230) is configured to be connected to a controlling organization externally owned account (235) configured to execute any blockchain transactions of the control client device (230) with the control smart contract (245) to modify the treatment data; and/or
the control smart contract (245) comprises a control smart contract data module (240) configured to store at least one of:
the treatment list of one or more treatments executable by each of the at least one treatment device (101);
the treatment recipes specifying each of the one or more treatments of the treatment list; and
pricing data associated with the execution of treatments by the treatment devices (101).

13. The system (100, 200) of any of claims 9 to 12, wherein the one or more smart contracts (220, 245) further comprise a physician smart contract (220) configured to be deployed on the blockchain network (170) to perform blockchain transactions; and wherein the system (100, 200) further comprises:
a physician client device (190) configured to execute a blockchain transaction with the blockchain network (170) via the physician smart contract (220) to manage prescription authorizations for each of the at least one treatment devices (101) to execute the one or more treatments of the treatment list;
wherein each treatment device (101) of the at least one treatment device (101) is further configured to interact with the physician smart contract (220) to determine whether the selected treatment from the treatment list has prescription authorization for being executed in the treatment device (101), wherein determining that the treatment device (101) has authorization to execute the selected treatment requires determining by the physician smart contract (220) that the treatment device (101) has prescription authorization to execute the selected treatment.

14. The system (100, 200) of claim 13, wherein the physician smart contract (220) comprises prescription authorization data indicating whether the selected treatment from the treatment list has prescription authorization for being executed in a treatment device (101);
wherein preferably the physician client device (190) is further configured to modify the prescription authorization data stored in the physician smart contract (220), and/or to replace the physician smart contract (220) with an updated physician smart contract (220) with updated prescription authorization data.

15. The system (100, 200) of any of claims 13 or 14, wherein:
the physician client device (190) is configured to be connected to a physician externally owned account (210) configured to execute any blockchain transactions of the physician client device (190) with the physician smart contract (220); and/or
the physician smart contract (220) comprises a physician smart contract data module (215) configured to store prescription authorization data.

16. The system (100, 200) of any of claims 9 to 15, wherein the blockchain network (170) is configured as:
an Ethereum-based network, and the system (100, 200) is configured such that, upon determination that the treatment device (101) has authorization to execute the selected treatment, this authorization is provided via smart contract code running on an Ethereum Virtual Machine, wherein preferably the blockchain network is a layer-2 chain configured to settle blockchain transactions on Ethereum; or
a Solana-based network, and the system (100, 200) is configured such that, upon determination that the treatment device (101) has authorization to execute the selected treatment, this authorization is provided via smart contract code running in a Solana Virtual Machine.

17. A method of authorizing a treatment device (101) of a system (100, 200) according to any of claims 9 to 17 to execute a selected treatment, the method comprising:
generating, by the treatment device (101), a request for receiving a treatment list from the blockchain network (170);
receiving, by the treatment device (101), from the blockchain network (170), a treatment list of one or more treatments suitable for being executed by the treatment device (101);
requesting, by the treatment device (101), authorization from the blockchain network (170) for executing a selected treatment from the treatment list;
determining, via the one or more smart contracts (220, 245) configured to be deployed on the blockchain network (170), whether the treatment device (101) has authorization to execute the selected treatment; and
receiving, by the treatment device (101), a recipe of the selected treatment upon determination that the treatment device (101) has authorization to execute the selected treatment;
wherein preferably the step of determining whether the treatment device (101) has authorization to execute the selected treatment comprises:
determining, preferably by the control smart contract (245), that a payment of a cost of the selected treatment has been successfully completed; and/or
determining, preferably by a physician smart contract (220), that the treatment device (101) has prescription authorization to execute the selected treatment.
